# FASCICULE DE BREVET EUROPEEN

(11) **EP 2 197 967 B1**
(45) Date de publication et mention de la délivrance du brevet: **30.11.2011**
(21) Numéro de dépôt: 08842343.9
(22) Date de dépôt: 01.10.2008
(51) Int. Cl.: C09D 151/08, A61K 9/28, C08L 5/00, C08L 51/08

(54) **COMPOSITION D'ENROBAGE COMPRENANT DU POLYDEXTROSE, PROCEDE POUR SA PREPARATION ET UTILISATION POUR ENROBER LES FORMES SOLIDES INGERABLES**
BESCHICHTUNGSZUSAMMENSETZUNG MIT POLYDEXTROSE, VERFAHREN ZU IHRER HERSTELLUNG UND IHRE VERWENDUNG ZUR BESCHICHTUNG EINNEHMBARER FESTSTOFFE
COATING COMPOSITION COMPRISING POLYDEXTROSE, PROCESS FOR PREPARING SAME AND USE FOR COATING INGESTIBLE SOLID FORMS

(30) Priorité: 05.10.2007 FR 0758091
(43) Date de publication de la demande: 23.06.2010
(73) Titulaire: Societe D'Exploitation De Produits Pour Les Industries Chimiques Seppic, 75007 Paris (FR)
(72) Inventeur: TROUVE, Gerard, F-81100 Castres (FR); LEFEBVRE, Sandra, F-81100 Castres (FR); MALANDAIN, Michel, F-78112 Fourqueux (FR)
(74) Mandataire: Grout de Beaufort, François-Xavier
(86) Numéro de dépôt international: PCT/FR2008/051773
(87) Numéro de publication internationale: WO 2009/053576

(56) Documents cités:
- US-A1- 2005 107 498

## Description

La présente invention a pour objet des compositions filmogènes destinées à l'enrobage coloré et brillant de formes solides ingérables, et plus particulièrement des comprimés pharmaceutiques destinés à un usage gastrique, les procédés de préparation des dites compositions filmogènes, les procédés d'enrobage mettant en oeuvre lesdites compositions filmogènes, et les produits pharmaceutiques enrobés colorés et brillants obtenus par la mise en oeuvre des procédés d'enrobage comprenant l'utilisation des dites compositions filmogènes. Le pelliculage de comprimés par des films de polymères colorés est couramment utilisé dans l'industrie pharmaceutique. L'amélioration de l'aspect extérieur de ces comprimés, en vue d'obtenir un aspect extérieur brillant constitue un enjeu économique. En effet, outre une considération purement esthétique qui permet de considérer qu'un comprimé brillant constitue un comprimé de qualité avancée, il est également avéré que la brillance des comprimés réduit l'appréhension des patients, qui ont des difficultés à avaler et à ingérer des comprimés, et permit par conséquent de renforcer le suivi médical des prescriptions. Par brillance on entend la résultante d'une des interactions de la lumière avec la matière. Lorsque la lumière frappe un objet, la plus grande partie de la lumière pénètre à l'intérieur de l'objet et une faible partie de la lumière est réfléchie à la surface de l'objet. Cette lumière de réflexion de surface est l'attribut d'apparence appelé « brillant ». Pour une surface brillante, l'angle du faisceau de la lumière réfléchie (dit « angle de miroir »), est égal à l'angle de la lumière incidente. Pour une surface mate, la lumière est réfléchie sous de nombreux angles au lieu du seul angle de miroir dans le cas de la surface brillante.

Il existe dans l'état de la technique des procédés d'enrobage et des produits de pelliculage qui permettent d'obtenir des films brillants. Les procédés de dragéification permettent d'obtenir des comprimés d'aspect brillant mais il s'agit de procédés peu économiques, qui consistent en la succession de plusieurs étapes consécutives. Les dragées sont des formes galéniques constituées d'un noyau central entouré d'une couche protectrice épaisse à base de sucre. Le noyau peut être une amande, un fruit sec, une pépite chocolatée pour les dragées utilisées en confiserie, ou bien un mini-comprimé contenant un ou des principes actifs médicamenteux pour les dragées utilisés en pharmacie. La couche protectrice est généralement constituée de sucre (saccharose) et d'une charge telle que le talc. Elle est formée par applications successives de sirop de sucre clair et de charges dans des turbines rotatives, chaque application de sirop et de charge étant suivie d'une étape de séchage. Les sirops de sucre sont obtenus par dissolution de saccharose dans l'eau à chaud sous agitation ; ils contiennent une concentration élevée de sucre, jusqu'à environ 70% à 80% en poids, afin de minimiser les quantités d'eau à évaporer. La coloration des dragées s'effectue au moyen d'un sirop de sucre coloré avec des pigments, des laques ou des colorants autorisés dans le domaine d'application. Ce sirop coloré est appliqué en fin de montage de la dragée, ce qui évite une coloration dans la masse onéreuse. Enfin, pour obtenir une dragée d'aspect brillant, il est procédé à une étape de lustrage ou de brillantage au moyen d'une dispersion de cire telle que la cire de carnauba ou la cire d'abeille. Ces procédés de montage et de brillantage de dragées sont connus depuis longtemps, sont décrits par exemple dans l'encyclopédie Kirk-Othmer (3ème édition, volume 17, page 281), et leur durée en font des procédés peu économiques. Les procédés de pelliculage sont plus rapides que les procédés de dragéification, car les quantités de film déposées sont plus faibles ; elles ne représentent que 3% à 5% de la masse de substrat enrobé. Ils imposent cependant la mise en oeuvre d'un équipement particulier consistant en un dispositif de pulvérisation associé à un système de séchage performant, car les quantités d'eau à évaporer sont importantes. Les procédés de pelliculage comprennent la préparation d'une dispersion, de préférence aqueuse, contenant un polymère filmogène, un système colorant, de préférence des pigments ou des laques, et éventuellement un plastifiant, des charges et/ou des additifs technologiques, puis sa pulvérisation sur les comprimés en mouvement dans une turbine rotative perforée ou dans un lit fluidisé. Un courant d'air chaud, entrant à une température supérieure ou égale à 40°C, assurent le séchage de la dispersion pulvérisée et provoquent la coalescence du film autour des comprimés. La couche déposée a une épaisseur de quelques dizaines de microns ; elle est opaque et colorée de manière homogène. Les polymères filmogènes les plus couramment utilisés sont des dérivés acryliques ou cellulosiques. Les polymères filmogènes acryliques sont principalement utilisés pour réaliser des formes pelliculées destinées à un usage entérique ou à un usage impliquant une libération prolongée du principe actif médicamenteux. Il se montrent cependant peu appropriés pour préparer des formes pelliculées brillantes destinées à être dissoutes dans un milieu gastrique. Les polymères filmogènes cellulosiques sont donc préférés pour la préparation de compositions de pelliculage destinées à préparer des formes pelliculées devant se dissoudre dans un milieu gastrique. Par polymère filmogène cellulosique, on désigne principalement les polymères comestibles choisis parmi les dérivés de la cellulose comme par exemple, les alkyl éthers de cellulose, les hydroxy alkyl éthers de cellulose, les esters monocarboxyliques de cellulose et les éthers mixtes de cellulose. Parmi ces produits, on peut citer les hydroxypropyméthyl celluloses (HPMC), les éthyl celluloses (EC), les méthyl celluloses (MC), les carboxyméthyl celluloses, les hydroxypropyl celluloses (HPC) ou les acétates ou phtalates de cellulose, tels que décrits par exemple dans la demande de brevet européen publiée sous le numéro EP 0 133 827 A1. On peut ainsi utiliser des polymères filmogènes cellulosiques déjà commercialisés sous forme de granulés comme, par exemple, de l'HPMC granulée connue sous le nom de PHARMA-COAT^{™} G. On peut encore utiliser des mélanges de polymères filmogènes cellulosiques différents comme par exemple les mélanges HPMC / EC. Le brevet US 6,468,561 enseigne également l'utilisation du polydextrose dans des compositions filmogènes, comprenant par exemple des polymères filmogènes cellulosiques, pour améliorer l'adhésion du film d'enrobage à la surface sur laquelle les compositions filmogènes sont appliquées et pour éliminer la sensation de mauvais goût dans la bouche des patients que procurent les autres dérivés de sucres habituellement utilisés. Cependant, les films obtenus par l'utilisation de tels polymères cellulosiques ne permettent pas d'obtenir un aspect brillant satisfaisant, et il est alors nécessaire d'ajouter une étape supplémentaire de lustrage de la forme pelliculée en utilisant une cire, induisant alors une augmentation significative du coût du procédé de pelliculage. Cette étape supplémentaire de lustrage peut être évitée par l'ajout dans la composition de pelliculage de pigments particuliers tels que les oxydes de titane, et plus particulièrement les Mica-Titanes, décrits par exemple dans la demande internationale publiée sous le numéro WO 2004/073582, commercialisés par la société Merck sous l'appellation Candurin^{™} Silver fine, Candurin^{™} Silver Luster, Candurin^{™} Red Shimmer, Candurin^{™} Sparkle Silvers. Cependant, l'effet visuel obtenu sur les formes pelliculées, préparées par la mise en oeuvre de compositions de pelliculage comprenant ces pigments, est un effet perlescent métallisé et non un effet brillant. Par perlescence on entend une couleur qui déploie différentes couleurs selon l'angle d'illumination et selon l'angle d'observation de la même manière que la nacre. La perlescence est calculée en mesurant le changement de couleur (DE) à des angles d'observation de 15° à une température de 25°C, par rapport à la réflexion spéculaire. La publication internationale WO 2004/073582 divulgue expressément un effet perlescent procuré par l'utilisation des pigments particuliers décrits précédemment et non un effet brillant. Par ailleurs, les polymères filmogènes cellulosiques forment rapidement des solutions visqueuses dans l'eau et ne peuvent être mis en oeuvre que dans des proportions massiques de 10% à 15% par rapport à 100% de la masse de la composition de pelliculage pour atteindre des solutions pulvérisables dans le procédé de pelliculage. L'utilisation de telles proportions massiques de polymères cellulosiques filmogènes nécessite alors l'évaporation de grandes quantités d'eau lors du procédé de pelliculage, ce qui en allonge la durée et en augmente le coût. Des polymères filmogènes induisant une moindre viscosité en solution aqueuse constitue une alternative à l'utilisation de copolymères filmogène cellulosiques pour préparer des formes pelliculées. Le brevet européen EP 1 124 541 B1 décrit l'utilisation de polymères greffés solubles dans l'eau ou qui se dispersent dans l'eau comme agents de revêtement, liants et/ou adjuvants filmogènes dans des formes galéniques pharmaceutiques. Ces copolymères greffés peuvent être obtenus par copolymérisation d'au moins un ester vinylique d'acides carboxyliques aliphatiques en C1-C24 en présence de polyéthers. La demande de brevet américain US 2005/0107498 A1 décrit des compositions de pelliculage de formes solides qui peuvent être facilement solubilisées dans l'eau ou facilement dispersées dans l'eau, induisant alors un temps réduit de préparation des solutions aqueuses ou des dispersions aqueuses de pelliculage qui peuvent être pulvérisées de façon aisée sans blocage des buses lors de l'étape de pulvérisation du procédé de préparation de la forme pelliculée. Ces compositions de pelliculage sont composées de copolymères greffés obtenus par la copolymérisation de l'alcool polyvinylique et de polyéthers, d'auxiliaires contenant des fonctions hydroxyles, amides ou esters, d'adjuvants de pelliculage. La composition de pelliculage décrite dans la demande US 2005/0107498 A1 ne résout pas complètement le problème posé car elle conduit à l'obtention de formes pelliculées d'aspect mat et non brillant.

C'est pourquoi les inventeurs ont cherché à développer de nouvelles compositions filmogènes, se solubilisant rapidement dans l'eau ou se dispersant rapidement dans l'eau, pour permettre la préparation de formes solides pelliculées destinées à un usage gastrique, d'aspect brillant, selon un procédé rapide et économique.

Selon un premier aspect, l'invention a pour objet Composition d'enrobage (C) comprenant pour 100% de sa masse :
- de 10% à 90% massique d'un copolymère greffé de l'alcool polyvinylique et de polyéthers (P₁), et
- de 90% à 10% massique d'un agent auxiliaire d'enrobage,
caractérisée en ce qu'une proportion massique non nulle dudit agent auxiliaire est le polydextrose et en ce que le rapport massique entre le copolymère greffé (P₁) et le polydextrose (P₂) est inférieur ou égal à 3.

Par copolymère greffé de l'alcool polyvinylique et de polyéthers (P₁), on désigne les copolymères obtenus par la copolymérisation :
- d'au moins un ester vinylique d'acides carboxyliques aliphatiques comprenant de un à 24 atomes de carbone, de préférence l'acétate de vinyle,
- avec au moins un polyéther de formule (I) :

   R₁-O-(R₂-O)ₓ-(R₃-O)_{y}-(R₄-O)_{z}-R₅ (I),
dans laquelle R₁ représente l'atome d'hydrogène, ou bien un radical alkyle, linéaire ou ramifié, comportant de 1 à 24 atomes de carbone, ou bien un radical acyle de formule R₆-C(=O)-, dans lequel R₆ est un radical hydrocarboné comportant de 1 à 23 atomes de carbone, ou bien un résidu de l'alcool polyvinylique. Selon un aspect particulier, R₁ est l'atome d'hydrogène ou le radical méthyle ;
R₅ représente l'atome d'hydrogène, ou bien un radical alkyle, linéaire ou ramifié, comportant de 1 à 24 atomes de carbone, ou bien un radical acyle de formule R₆-C(=O)-, dans lequel R₆ est un radical hydrocarboné comportant de 1 à 23 atomes de carbone, R₅ représentant plus particulièrement l'atome d'hydrogène.

R₂, R₃ et R₄ identiques ou différents, représentent indépendamment l'un des autres, un radical, choisi parmi les groupes de formules :
- -(CH₂)ₙ- pour lesquels n est nombre entier compris entre 2 et 4 ;
- -CH₂-CH(CH₃)-
- -CH₂-CH(CH₂-CH₃)-
- -CH₂-CH-OR₇-CH₂- pour lequel R₇ représente l'atome d'hydrogène, ou bien un radical alkyle, linéaire ou ramifié, comportant de 1 à 24 atomes de carbone, ou bien un radical acyle de formule R₆-C(=O)-, dans lequel R₆ est un radical hydrocarboné comportant de 1 à 23 atomes de carbone ; R₂, R₃ et R₄ étant préférentiellement choisis parmi
- (CH₂)₂- et -CH₂-CH(CH₃)- ;
- x est un nombre compris entre 1 et 5000, plus particulièrement entre 10 et 2000, et tout particulièrement entre 20 et 500 ;
- y est un nombre compris entre 0 et 5000,
- z est un nombre compris entre 0 et 5000,
- y et z étant préférentiellement choisis égaux à 0 lorsque x est supérieur à 10.

Le calcul du poids moléculaire du polyéther de formule (I) à partir des valeurs de x, y et z, permet d'obtenir une valeur moyenne, bien que des tels produits possèdent une distribution large du poids moléculaire.

Les polyéthers de formule (1) préférentiellement utilisés sont ceux dont le poids moléculaire moyen est compris entre 400 et 50 000 g.mol⁻¹, et plus préférentiellement ceux dont le poids moléculaire moyen est compris entre 1 500 et 20 000 g.mol⁻¹.

Les groupements esters polyvinyliques des copolymères greffés de l'alcool polyvinylique et de polyéthers sont par la suite hydrolysés en toute ou partie.

La préparation de tels copolymères greffés est décrite par exemple dans les demandes de brevet allemand publiées sous les numéros DE 1 077 430, DE 1 094 457 et DE 1 081 229. De tels copolymères greffés sont par exemples commercialisés sous l'appellation Kollicoat^{™}.

Par agent auxiliaire d'enrobage, on désigne les composés ou les mélanges de composés solubles dans l'eau ou qui se dispersent dans l'eau, choisis parmi le groupe comprenant des polymères contenant des fonctions hydroxyles, amides ou esters, et des composés organiques de faible masse moléculaire. Les agents auxiliaires d'enrobage sont conformes aux réglementations en vigueur dans les domaines pharmaceutique, diététique ou alimentaire.

Les polymères contenant des fonctions hydroxyles, amides ou esters utilisés comme agents auxiliaires d'enrobage sont en général choisis parmi :
- les alcools polyvinyliques, les polysaccharides, les celluloses, les amidons, les polymères de l'acide lactique, les polyéthylène-glycols, les polypropylène-glycols ou les copolymères blocs comprenant des polyéthylène-glycols et des polypropylène-glycols, ainsi que leurs dérivés ;
- les polyvinyl pyrrolidones, les copolymères de la vinyl pyrrolidone et de l'acétate vinylique ;
- les copolymères de la vinyl pyrrolidone et de l'acide méthacrylique, les copolymères de la vinyl pyrrolidone et de l'acide acrylique ;
- les copolymères (méth)acryliques, les copolymères hydroxyalkyl (méth)acryliques comme ceux décrits dans la demande de brevet allemand publiée sous le numéro
   DE 1 004 9297, les acétates polyvinyliques ; et
- la gélatine.

Selon un aspect particulier de la présente invention, les polymères contenant des fonctions hydroxyles, amides ou esters utilisés comme agents auxiliaires d'enrobage, sont choisis parmi les alcools polyvinyliques ayant un taux d'hydrolyse compris entre 80% et 99% molaire, les hydroxypropylméthyl celluloses (HPMC), les hydroxypropyl celluloses (HPC), les hydroxyéthylméthyl celluloses, les méthyl celluloses, les carboxyméthyl celluloses de sodium, l'amylose, les maltodextrines, les sirops de glucose, les cyclodextrines, les dextranes, l'inuline, les polyfructose, les alginates tels que l'alginate de propylène glycol, les pectines, les carraghénates, le guar, les gommes xanthanes, les gommes arabiques, les terpolymères du méthacrylate de butyle et du méthacrylate de 2-(diméthylamino) éthyle et du méthacrylate de méthyle, les copolymères de l'acide méthacrylique et de l'acrylate d'éthyle, les chitosanes, les éthyl celluloses, les polyvinyl pyrrolidones réticulées, l'acétate de polyvinyle, les celluloses et plus particulièrement les cellulose microcristallines.

Le polydextrose utilisé en une proportion massique non nulle dudit agent auxiliaire d'enrobage, est un polysaccharide hydrosoluble obtenu par la polymérisation du glucose, ou dextrose, dont les caractéristiques et les modes de préparation sont décrits dans les brevets américains publiés sous les numéros US 3,766,165 et US 3,876,794. Le polydextrose est disponible dans le commerce.

Les composés organiques de faible masse moléculaire utilisés comme agents auxiliaires d'enrobage sont en général choisis parmi l'urée, les sucres, les alcools dérivés de sucres, les dérivés de sucres ou d'alcools dérivés de sucres, les silices dont la surface spécifique est supérieure ou égale à 100 m²g⁻¹. Les composés organiques de faible masse moléculaire utilisés comme agents auxiliaires d'enrobage préférés sont le lactose, le sucrose, le glucose, le mannitol, le sorbitol, l'isomalt, le xylitol.

Selon un aspect particulier, la composition d'enrobage (C) telle que définie précédemment comprend en outre pour 100% de sa masse, de 2% à 30% massique d'un agent plastifiant ou d'un mélange d'agents plastifiants.

Par agent plastifiant, on entend principalement soit les agents plastifiants comestibles solubles comme les polyols en général et, plus particulièrement le sorbitol, le mannitol, le xylitol, le glycérol, le saccharose, les polyéthylène glycols, les propylène glycols, soit les agents plastifiants comestibles peu hydrosolubles tels que ceux comprenant une chaîne aliphatique comprenant au moins 12 atomes de carbone, par exemple, l'acide stéarique, les sels de l'acide stéarique, comme le stéarate de magnésium, le stéarate d'aluminium, l'acide stéarique polyéthoxylé, les monoglycérides d'acides gras, les diglycérides d'acides gras et leurs dérivés estérifiés par l'acide acétique, l'acide tartrique ou l'acide lactique, les esters d'acides gras et de propylène glycol, les esters d'acides gras et de sorbitol, les esters d'acides gras et de sorbitan, les esters d'acides gras et de mannitol, les esters d'acides gras et de mannitan ou encore certains sucroesters, les sucroglycérides ou les esters de polyglycérol, en particulier ceux caractérisés par un nombre HLB inférieur à 7.

Selon un autre aspect particulier, la composition d'enrobage (C) telle que définie précédemment, comprend en outre pour 100% de sa masse de 2% à 30% massique d'un ou plusieurs agents de coloration.

Par agent de coloration utilisés dans l'invention on désigne principalement les colorants ou les pigments, comme par exemple ceux cités dans les pharmacopées européennes ou américaines, ou dans les listes d'additifs alimentaires, référencés en Europe sous les numéros E 100 à E 172, comme, par exemple, les oxydes de fer, les oxydes de titane, de zinc ou de magnésium, les colorants absorbés sur laques d'alumines, les mica-titane, ou certains colorants naturels comme le caramel, les caroténoïdes, la riboflavine, l'indigotine, la carmine ou la chlorophylle. On peut aussi avantageusement utiliser des colorants composites constitués d'un assemblage de silicates de potassium et d'aluminium (mica), de dioxyde de titane et de colorants, tels que ceux commercialisés par la société Merck sous le nom de Candurin^{™}.

Selon un autre aspect particulier, la composition d'enrobage (C) telle que définie précédemment, comprend en outre pour 100% de sa masse de 2% à 30% massique d'une ou plusieurs charges inertes.

Les charges inertes utilisées dans l'invention sont celles qui permettent de modifier les propriétés du matériau d'enrobage et la protection dudit matériau. A titre d'exemple de charges inertes, on mentionnera les agents opacifiants comme par exemple le talc, le kaolin, l'oxyde de titane ; les agents tensioactifs mouillants et dispersants de qualité alimentaire comme par exemple les esters de sorbitan, les esters de sorbitan éthoxylés, les huiles de ricin hydrogénées éthoxylées, le lauryl sulfate de sodium, les alcools gras éthoxylés, les acides gras éthoxylés ; les agents anti-mousses et les agents réducteurs de mousses tels que les acides gras , les silicones, les dérivés de silicone ; les agents diluants comme par exemple le lactose, le sucrose, le mannitol, le xylitol, l'isomalt, le talc ; l'hydrogénophosphate de calcium ; le stéarate de magnésium ; le monostéarate de glycérol.

Selon un autre aspect particulier, la composition (C) telle que définie précédemment, comprend en outre de 1% massique à 20% massique d'un agent facilitant l'écoulement, comme par exemple une silice colloïdale finement divisée telle que celles commercialisées par la société Degussa sous l'appellation « Acrosil^{™} ». Les agents auxiliaires d'enrobage tels que définis ci-dessus, présentent parfois eux même, la propriété de favoriser l'écoulement des compositions d'enrobages sous forme de poudres ou de granulés et il n'est dans ce cas pas nécessaire d'en ajouter un autre.

Selon un autre aspect particulier de la présente invention, le rapport massique entre le copolymère greffé (P1) et le polydextrose mis en oeuvre dans la composition d'enrobage (C) est supérieur ou égal à 1/3, et plus particulièrement supérieur ou égal à 2/3.

Selon un autre aspect particulier de la présente invention, le rapport massique entre le copolymère greffé (P1) et le polydextrose mis en oeuvre dans la composition d'enrobage (C) est inférieur ou égal à 3, et plus particulièrement inférieur ou égal à 3/2.

Comme exemples de compositions d'enrobage (C), il y a les compositions pour lesquelles le rapport massique entre le copolymère greffé (P1) et le polydextrose est compris entre 2/3 et 3/2.

Les tests comparatifs ci-dessous montrent que lorsque ce rapport massique est supérieur à 3 (il vaut 4,5 pour le produit 2 et 7 pour le produit 3, voir tableau A), la brillance est très inférieure à celle obtenue pour une composition dont ce rapport est compris entre 1/3 et 3 (il vaut 1,17 pour le produit 1), comme cela est résumé dans le tableau B.

### Tests comparatifs

**Tableau A**

| | Produit 1 | Produit 2 | Produit 3 |
|---|---|---|---|
| Copolymère PVA-PEG (P₁) | 35% | 45% | 70% |
| Polydextrose (P₂) | 30% | 10% | 10% |
| Kaolin | 20% | 30% | |
| Pigments (oxyde de fer rouge + TiO2) | 15% | 15% | 20% |
| % solides dans la solution de pelliculage | 25% | 25% | 25% |
| | | | |
| Rapport massique P₁/P₂ | 1,17 | 4,5 | 7 |

### - Préparation des dispersions

Les produits de pelliculage sont mis en dispersion à 25% dans l'eau. Pour chaque produit, 600g de dispersion sont préparés : 150g de produit sont dispersés dans 450g d'eau purifiée à température ambiante. La mise en dispersion est réalisée à l'aide d'un agitateur de laboratoire type Turbotest V2004 (Rayneri) et d'une turbine défloculeuse. La vitesse d'agitation est ajustée de manière à éviter d'incorporer de l'air dans la dispersion, ce qui permet de ne pas former de mousse. Après 45 minutes d'agitation, les dispersions sont prêtes.

### - Pelliculage

Les dispersions sont pulvérisées sur des comprimés placebos dans une turbine de pelliculage perforée Driacoater 500 (charge = 3 kg de noyaux). Les conditions opératoires suivantes sont suivies : débit d'air=300m³/h- température d'entrée de l'air de séchage = 55°C-60°C. La température des noyaux varie entre 36°C-38°C pendant le pelliculage. Un dépôt sec théorique de 3% est appliqué sur les comprimés.

**Tableau B**

| | Produit 1 | Produit 2 | Produit 3 |
|---|---|---|---|
| Densité de brillance | 990 | 80 | 130 |

Selon un autre aspect particulier, l'invention a pour objet une composition d'enrobage (C) telle que définie précédemment, caractérisée en ce qu'elle se présente sous une forme prête à l'emploi contenant les mélanges de ses différents constituants sous la forme d'une dispersion aqueuse, d'une poudre ou de granulés prêts à l'emploi.

Par dispersion aqueuse, on entend les dispersions réalisées dans l'eau ou les mélanges d'eau et d'alcools hydrosolubles comme par exemple l'éthanol.

Les compositions prêtes à l'emploi présentent plusieurs avantages :
- La manipulation, le stockage, le contrôle d'un seul et unique produit ;
- Une meilleure reproductibilité des couleurs et des performances ;
- Une mise en dispersion plus aisée.

L'invention a aussi pour objet un procédé de préparation d'une composition d'enrobage (C) telle que définie précédemment et se présentant sous la forme d'une poudre sèche, comprenant les étapes suivantes :
une étape (a) de mélange du copolymère greffé (P₁), du polydextrose et, si nécessaire ou si désiré un ou plusieurs autres agents auxiliaires d'enrobage, un ou plusieurs agents plastifiant, un ou plusieurs agents de coloration, une ou plusieurs charges inertes et/ou un ou plusieurs agents facilitant l'écoulement,
une étape (b) de broyage du mélange issue de l'étape (a) pour former ladite composition (C).

Dans le procédé tel que défini précédemment, pour la mise en oeuvre de l'étape (a), l'ensemble des composants est ajouté de manière séquencée ou simultanée. Le mélange est ensuite généralement effectué avec un dispositif de type mélangeur à barre.

L'étape (b) du procédé tel que défini ci-dessus est par exemple effectué au moyen d'un broyeur à couteaux de façon à obtenir une poudre finement divisées ou avec un dispositif de cryo-broyage généralement sous azote liquide. Un tel dispositif permet d'optimiser la granulométrie finale de la composition d'enrobage (C).

L'invention a aussi pour objet un procédé de préparation d'une composition d'enrobage (C) telle que définie précédemment et se présentant sous la forme de granulés prêts à l'emploi, comprenant les étapes suivantes :
une étape (a1) de mouillage du copolymère greffé (P₁) et du polydextrose, avec éventuellement si nécessaire ou si désiré un ou plusieurs autres agents auxiliaires d'enrobage, un ou plusieurs agents plastifiant, un ou plusieurs agents de coloration, une ou plusieurs charges inertes et/ou un ou plusieurs agents facilitant l'écoulement, par une solution liante, afin d'obtenir une masse humide contenant de 30 à 60% d'eau,
une étape (b1) de séchage de la masse humide obtenue à l'étape (a1), et si désiré ou si nécessaire,
une étape (c1) de calibrage de la masse séchée obtenue à l'étape (b1) pour obtenir ladite composition (C).

Le procédé tel que défini ci-dessus est par exemple décrit dans les demandes de brevet français publiées sous les numéros FR 2 548 675 et FR 2 660 317. ou dans l'encyclopédie Kirk Othmer (3ème édition volume 17, page 281).

Par granulés, on entend principalement des agglomérats de plusieurs dizaines à plusieurs milliers de particules de matière, initialement individualisées, pouvant être de nature identique ou différente.

Les étapes (a1) et (b1) du procédé tel que défini ci-dessus, sont notamment effectuées dans un mélangeur-granulateur ou dans un lit fluidisé.

L'étape (c1) du procédé tel que défini ci-dessus, est notamment effectuée dans une étuve ou dans un lit fluidisé.

L'invention a aussi pour objet un procédé de préparation d'une composition d'enrobage (C) telle que définie précédemment et se présentant sous la forme de granulés prêts à l'emploi, comprenant les étapes suivantes :
- une étape (a2) d'homogénéisation du copolymère greffé (P₁) et du polydextrose, avec si nécessaire ou si désiré un ou plusieurs autres agents auxiliaires d'enrobage, un ou plusieurs agents plastifiant, un ou plusieurs agents de coloration, une ou plusieurs charges inertes et/ou un ou plusieurs agents facilitant l'écoulement, dans un lit fluidisé par soufflage d'air,
- une étape (b2) de pulvérisation progressive d'eau du mélange fluidisé résultant de l'étape (a2) jusqu'à former une masse humide, et
- une étape (c2) de séchage de la masse humide résultant de l'étape (b2) par soufflage d'air chaud, pour obtenir ladite composition (C).

Dans le procédé tel que défini ci-dessus l'air chaud est généralement à une température comprise entre 70°C et 100°C.

Un tel procédé est par exemple décrit dans la demande de brevet publiée sous le numéro EP 1 552 819.

L'invention a aussi pour objet l'utilisation de la composition d'enrobage (C), telle que définie précédemment, pour enrober des formes solides ingérables.

Par forme solide ingérable, on désigne les formes solides ingérables par l'homme ou l'animal et quelle qu'en soit leur destination, qu'il s'agisse de médicaments, de compléments alimentaires, de formes à visée cosmétique, de confiserie ou de sucrerie. L'utilisation de la composition d'enrobage (C), telle que définie précédemment, est plus particulièrement destinées aux comprimés.

L'invention a aussi pour objet un procédé d'enrobage de formes solides comestibles, comprenant :
- une étape (a3) de dispersion :
   - Où bien du copolymère greffé (P₁), du polydextrose et, si nécessaire ou si désiré, d'un ou de plusieurs autres agents auxiliaires d'enrobage, d'un ou de plusieurs agents plastifiant, d'un ou de plusieurs agents de coloration, d'une ou de plusieurs charges inertes et/ou d'un ou de plusieurs agents facilitant l'écoulement, dans un solvant adapté tel qu'un milieu aqueux
   - Où bien de la composition (C) telle que définie précédemment, pour former une dispersion (D₁);
- une étape (b3) de pulvérisation de la dispersion (D₁) obtenue à l'étape (a3) dans laquelle les solutions ou les dispersions ainsi obtenues lors de l'étape (a3) sont pulvérisées sur des substrats solides à enrober.

Dans l'étape (a3) du procédé tel que défini ci-dessus, les différents constituants de sont maintenus en dispersion à l'aide d'un agitateur et d'une turbine défloculeuse, tout en évitant la formation de mousse,

Dans l'étape (a3) du procédé tel que défini ci-dessus, la composition d'enrobage (C) représente entre 6% et 30% de 100% de la masse de ladite dispersion (D₁).

L'invention a enfin pour objet la dispersion (D1) obtenue à l'étape a3 du procédé tel que défini ci-dessus.

La mise en oeuvre de la composition d'enrobage (C), selon les procédés précédemment décrits, permet de préparer des produits pharmaceutiques enrobés, destinés à un usage gastrique et présentant un aspect brillant. Les exemples suivants illustrent l'invention sans toutefois la limiter.

### A- Préparation de compositions d'enrobage selon l'invention.

### Composition (C₁)

On prépare une composition d'enrobage (C₁), en ajoutant à température ambiante, successivement, 48 g d'un copolymère greffé (P₁) d'alcool polyvinylique et de Polyéthylène glycol (PVA/PEG), commercialisé sous l'appellation Kollicoat^{™} IR par la société BASF, 30 g de polydextrose, 24 g de kaolin et 18 g d'un mélange d'oxyde de fer rouge et d'oxyde de titane, dans 480 g d'eau purifiée à une température de 25°C. La mise en dispersion est réalisée à l'aide d'un agitateur de laboratoire type Turbotest^{™} V2004 (Rayneri) et d'une turbine défloculeuse. La vitesse d'agitation est ajustée de manière à éviter d'incorporer de l'air dans la dispersion, pour ne pas former de mousse. Après 45 minutes d'agitation, on obtient 600 g de composition (C₁) sous forme d'une dispersion aqueuse, dans laquelle la proportion massique en solides en représente 20%.

### Composition (C₂)

On prépare une composition d'enrobage (C₂), en ajoutant à température ambiante, successivement 36 g de Kollicoat^{™} IR, 30 g de polydextrose, 12 g d'hydroxypropyméthyl cellulose (HPMC) 6 mPas, 24 g de kaolin et 18 g d'un mélange d'oxyde de fer rouge et d'oxyde de titane dans 480 g d'eau purifiée. La mise en dispersion est réalisée à l'aide d'un agitateur de laboratoire type Turbotest^{™} V2004 et d'une turbine défloculeuse. La vitesse d'agitation est ajustée de manière à éviter d'incorporer de l'air dans la dispersion, pour ne pas former de mousse. Après 45 minutes d'agitation, on obtient la composition (C₂) sous forme d'une dispersion aqueuse, dans laquelle la proportion massique en solides en représente 20%.

### Composition (C₃)

On prépare une composition d'enrobage (C₃), en ajoutant à température ambiante, successivement 36 g de Kollicoat^{™} IR, 42 g de polydextrose, 19,2 g de kaolin, 4,8 g d'un polyéthylène glycol de poids moléculaire moyen de 400 (PEG 400) et 18 g d'un mélange d'oxyde de fer rouge et d'oxyde de titane dans 480 g d'eau purifiée. La mise en dispersion est réalisée à l'aide d'un agitateur de laboratoire type Turbotest^{™} V2004 et d'une turbine défloculeuse. La vitesse d'agitation est ajustée de manière à éviter d'incorporer de l'air dans la dispersion, pour ne pas former de mousse. Après 45 minutes d'agitation, on obtient la composition (C₃) sous forme d'une dispersion aqueuse, dans laquelle la proportion massique en solides en représente 20%.

### Composition (C₄)

On prépare une composition d'enrobage (C₄), en ajoutant à température ambiante, successivement 45 g de Kollicoat^{™} IR, 75 g de polydextrose, 7,5 g de kaolin, et 22,5 g d'un mélange d'oxyde de fer rouge et d'oxyde de titane dans 450 g d'eau purifiée. La mise en dispersion est réalisée à l'aide d'un agitateur de laboratoire type Turbo-test^{™} V2004 et d'une turbine défloculeuse. La vitesse d'agitation est ajustée de manière à éviter d'incorporer de l'air dans la dispersion, pour ne pas former de mousse. Après 45 minutes d'agitation, on obtient la composition (C₄) sous forme d'une dispersion aqueuse, dans laquelle la proportion massique en solides en représente 25%.

### Composition (C₅)

On prépare une composition d'enrobage (C₅), en ajoutant à température ambiante, successivement 27 g de Kollicoat^{™} IR, 45 g de polydextrose, 4,5 g de kaolin, et 13,5 g d'un mélange d'oxyde de fer rouge et d'oxyde de titane dans 510 g d'eau purifiée. La mise en dispersion est réalisée à l'aide d'un agitateur de laboratoire type Turbo-test^{™} V2004 et d'une turbine défloculeuse. La vitesse d'agitation est ajustée de manière à éviter d'incorporer de l'air dans la dispersion, pour ne pas former de mousse. Après 45 minutes d'agitation, on obtient la composition (C₅) sous forme d'une dispersion aqueuse, dans laquelle la proportion massique en solides en représente 15%.

### B - Préparation de compositions d'enrobage selon l'état de la technique.

### Composition (T₁)

On prépare une composition d'enrobage (T₁), en ajoutant à température ambiante, successivement 48 g de Kollicoat^{™} 1R, 30 g de cellulose microcristalline, 24 g de kaolin et 18 g d'un mélange d'oxyde de fer rouge et d'oxyde de titane dans 480 g d'eau purifiée. La mise en dispersion est réalisée à l'aide d'un agitateur de laboratoire type Turbotest^{™} V2004 et d'une turbine défloculeuse. La vitesse d'agitation est ajustée de manière à éviter d'incorporer de l'air dans la dispersion, pour ne pas former de mousse. Après 45 minutes d'agitation, on obtient la composition (T₁) sous forme d'une dispersion aqueuse, dans laquelle la proportion massique en solides en représente 20%.

### Composition (T₂)

On prépare une composition d'enrobage (T₂), en ajoutant à température ambiante, successivement 48 g d'HPMC (6 mPas), 42 g de cellulose microcristalline, 12 g d'acide stéarique éthoxylé à 40 moles d'oxyde d'éthylène, et 18 g d'un mélange d'oxyde de fer rouge et d'oxyde de titane dans 480 g d'eau purifiée. La mise en dispersion est réalisée à l'aide d'un agitateur de laboratoire type Turbotest^{™} V2004 et d'une turbine défloculeuse. La vitesse d'agitation est ajustée de manière à éviter d'incorporer de l'air dans la dispersion, pour ne pas former de mousse. Après 45 minutes d'agitation, on obtient la composition (T₂) sous forme d'une dispersion aqueuse, dans laquelle la proportion massique en solides en représente 20%.

### Composition (T₃)

On prépare une composition d'enrobage (T₃) en ajoutant à température ambiante, successivement 66 g de Kollicoat^{™} IR, 8 g d'HPMC (6 mPas), 24 g de kaolin, 2,4 g de cellulose microcristalline et 18 g d'un mélange d'oxyde de fer rouge et d'oxyde de titane dans 480 g d'eau purifiée. La mise en dispersion est réalisée à l'aide d'un agitateur de laboratoire type Turbotest^{™} V2004 et d'une turbine défloculeuse. La vitesse d'agitation est ajustée de manière à éviter d'incorporer de l'air dans la dispersion, pour ne pas former de mousse. Après 45 minutes d'agitation, on obtient la composition (T₃) sous forme d'une dispersion aqueuse, dans laquelle la proportion massique en solides en représente 20%.

### Composition (T₄)

On prépare une composition d'enrobage (T₄) en ajoutant à température ambiante, successivement 23,04 g de carboxymethylcellulose de sodium (Na CMC), 9,12 g de maltodextrine, 4,8 g de dextrose, 3,84 g de lécithine, 7,2 g d'un mélange d'oxyde de fer rouge et d'oxyde de titane dans 552 g d'eau purifiée à une température de 25°C. La mise en dispersion est réalisée à l'aide d'un agitateur de laboratoire type Turbotest^{™} V2004 et d'une turbine défloculeuse. La vitesse d'agitation est ajustée de manière à éviter d'incorporer de l'air dans la dispersion, pour ne pas former de mousse. Après 45 minutes d'agitation, on obtient la composition (T₄) sous forme d'une dispersion aqueuse, dans laquelle la proportion massique en solides en représente 8%.

### C - Préparation de formes solides enrobées.

Les compositions d'enrobage C₁, C₂, C₃, C₄ et C₅ selon l'invention et T₁, T₂, T₃ et T₄ de l'état de la technique, sont pulvérisées sur 3 kg de comprimés placebos placés dans une turbine de pelliculage perforée de marque Dricoater^{™} 500. Le débit d'air de séchage circulant dans la turbine est fixé à 300 m³.h⁻¹, la température d'entrée dans la turbine de l'air de séchage est fixée à une température comprise entre 55°C et 60°C. La température des noyaux varie dans l'opération de préparation de pelliculage entre 36°C et 38°C. Un dépôt sec théorique de 3% massique pour 100% de la masse du comprimé est appliqué sur les comprimés.

Les compositions d'enrobage C₁, C₁, C₃, C₄ et C₅ selon l'invention et T₁, T₂, T₃ et T₄ de l'état de la technique, préparées dans les exemples précédents, sont utilisées pour préparer respectivement les comprimés enrobés E_{C1}, E_{C2}, E_{C3}, E_{C4}, E_{C5} selon l'invention et E_{T1}, E_{T2}, E_{T3}, et E_{T4} selon l'état de la technique.

### D - Mesure de la brillance des comprimés enrobés.

### 1 - A l'aide d'un brillance-mètre.

Les brillance-mètres sont utilisés pour réaliser la mesure de brillance des comprimés pelliculés. Ils reposent sur le principe consistant à projeter un rayon lumineux sur la surface à analyser, et à mesurer l'intensité de la lumière réfléchie sous un angle symétrique à l'angle d'incidence.

Des appareils dédiés à la mesure de brillance sur des surfaces courbes comme la surface des comprimés sont également disponibles. Ils reposent sur le même principe de mesure que précédemment décrit, en utilisant cependant un système d'imagerie vidéo de haute résolution. Lors de la mesure, chaque comprimé est éclairé par un éclairage puissant type lumière du jour. La lumière réfléchie par le comprimé est analysée par imagerie et décomposée en :
- réflexion spéculaire (qui donne l'effet brillant),
- réflexion diffuse (qui donne la couleur).

### 2 - A l'aide d'une méthode visuelle

Les comprimés enrobés dont on cherche à évaluer la brillance sont disposé côte à côte dans un récipient ouvert placé sous la lumière naturelle du jour. Un panel représentatif de 10 utilisateurs dûment entraînés à cette évaluation, examine les comprimés enrobés disposés dans les mêmes conditions. Chaque membre du panel choisit de façon indépendante, sans communiquer son choix aux autres membres du panel, le comprimé enrobé qui lui semble présenter l'aspect brillant le plus intense. Chaque membre du panel ne dispose qu'une seule possibilité de choix. Le recueil des choix est ensuite réalisé par de façon à déterminer les comprimés enrobés qui ont recueillis le plus grand nombre de choix de la part des membres du panel.

### 3 - Résultats obtenus d'un brillance-mètre

La brillance des comprimés enrobés E_{C1}, E_{T1}, E_{T2}, E_{C2}, E_{C3} et E_{T3}, exprimée par son taux de brillance, est évaluée selon le principe décrit au paragraphe 1 précédent, à l'aide d'une caméra numérique haute résolution. Les résultats obtenus sont reportés dans le tableau 1 ci-dessous :

**Tableau 1**

| Comprimé enrobé | E_{C1} | E_{T1} | E_{T2} | E_{C2} | E_{C3} | E_{T3} |
|---|---|---|---|---|---|---|
| Taux de brillance | 0.77 | 0.61 | 0.58 | 0.77 | 0.80 | 0.60 |
| Densité de brillance | 1707 | 705 | 1188 | | | |

Ils mettent en évidence que le comprimé E_{C1}, enrobé à l'aide de la composition C₁, caractérisée par un rapport massique entre le copolymère greffé (PVA/PEG) et le polydextrose de 1,6, possède un taux de brillance de 0,77, en augmentation de 26,2 % par rapport au taux de brillance du comprimé E_{T1} enrobé à l'aide de la composition T₁ exempte de polydextrose, et en augmentation de 32,8% par rapport au taux de brillance au taux de brillance du comprimé E_{T2} enrobé à l'aide de la composition T₃ exempte à la fois de polydextrose et de copolymère greffé (PVA/PEG).

De même, les comprimés E_{C2} et E_{C3}, enrobés à l'aide des compostions C₂ et C₃ caractérisées par des rapports massiques entre le copolymère greffé (PVA/PEG) et le polydextrose respectifs de 1,2 et de 0,86, possèdent les taux de brillance respectifs de 0,77 et de 0,80, en augmentation respective de 28,3% et de 33,3% par rapport au comprimé E_{T3} enrobé à partir de la composition T₃ exempte de polydextrose.

### 4 - Résultats obtenus par la méthode visuelle

**(i)**- La brillance des comprimés enrobés E_{C1} et E_{T1}, exprimée par son taux de sélection par les membres d'un panel de 10 personnes dûment entraînés, est évaluée selon le principe décrit dans au paragraphe 2. Les résultats obtenus sont reportés dans le tableau 2 ci-dessous :

**Tableau 2**

| Comprimé enrobé | E_{C1} | E_{T1} |
|---|---|---|
| % de choix des comprimés enrobés jugés les plus brillants | 95,0% | 5,0% |

Le comprimé E_{C1} enrobé à l'aide de la composition C₁, qui se caractérise par un rapport massique entre le copolymère greffé (PVA/PEG) et le polydextrose de 1,6, a été jugé comme étant le comprimé le plus brillant par 95% des membres du panel, alors que le comprimé E_{T1}, enrobé à l'aide de la composition T₁ ne comprenant pas de polydextrose, a été sélectionné par seulement 5% des membres du panel comme étant le comprimé enrobé le plus brillant.
**(ii)-** La brillance des comprimés enrobés E_{C4}, E_{C5} et E_{T4}, exprimée par son taux de sélection par les membres du panel de 10 personnes dûment entraînés, est évaluée selon le principe décrit au paragraphe 2 précédent. Les résultats obtenus sont reportés dans le tableau 3 ci-dessous :

**Tableau 3 :**

| Comprimé enrobé | E_{C4} | E_{C5} | E_{T4} |
|---|---|---|---|
| % de choix des comprimés enrobés jugés les plus brillants | 43,75 % | 31,25 % | 25,0% |

Les comprimés E_{C4} et E_{C5} enrobés respectivement à l'aide des compositions C₄ et C₅, caractérisées chacune par un rapport massique identique entre le copolymère greffé (PVA/PEG) et le polydextrose de 0,6, ont été jugés comme étant les comprimés les plus brillants respectivement par 43,75% et 31,25% des membres du panel ou, de façon cumulative par 75% des membres du panel comme étant les composés les plus brillants alors que le comprimé E_{T4} enrobé à l'aide de la composition T₄ comprenant un autre polysaccharide, la maltodextrine et un éther de cellulose, n'a été jugée comme étant la plus brillante que par 25% du panel.

### Méthode de mesure de la densité de brillance utilisée pour toute les mesures :

Afin de mettre en évidence la densité de brillance d'un échantillon, celui-ci est illuminé par une source directive sous un angle donné. Une caméra permet alors d'obtenir des images. Les zones qui présentent un fort taux de brillant sont ainsi localisées.

Cette méthode de cartographie permet de représenter la distribution spatiale du brillant. Nous pouvons en extraire la densité de brillance = nombre de pixels/ mm².

### Autres tests comparatifs :

Le tableau ci-dessous met en valeur les résultats de quatre essais comparatifs.
L'essai 1 met en oeuvre une composition comprenant du Kollicoat (P1) et de la dextrine (P2) dans un rapport P1/P2 = 0,86 (donc inférieur à 3).
L'essai 2 met en oeuvre une composition comprenant du Kollicoat (P1) et de l'Isomalt (P2) dans un rapport P1/P2 = 0,86 (donc inférieur à 3).
L'essai 3 met en oeuvre une composition conforme à la présente invention comprenant du Kollicoat (P1) et du polydextrose (P2) dans un rapport P1/P2 = 0,86 (donc inférieur à 3).
L'essai 4 met en oeuvre une composition comprenant du Kollicoat (P1) et de la dextrine (P2) dans un rapport P1/P2 = 4 (donc supérieur à 3).

| | Brillance |
|---|---|
| **Essai 1** | |
| Kollicoat IR=30% | |
| Dextrine=35% | **Densité de brillance : 267** |
| Kaolin=18% | |
| PEG 400=2% | |
| Pigments=15% | |
| **Essai 2** | |
| Kollicoat IR=30% | |
| Isomalt=35% | **Densité de brillance : 822** |
| Kaolin=18% | |
| PEG 400=2% | |
| Pigments=15% | |
| **Essai 3** | |
| Kollicoat IR=30% | |
| Polydextrose=35% | **Densité de brillance : 1521** |
| Kaolin=18% | |
| PEG 400=2% | |
| Pigments=15% | |
| **Essai 4** | |
| Kollicoat IR=60% | |
| Dextrine=15% | **Densité de brillance : 799** |
| HPMC=10% | |
| Glycérine=5% | |
| Pigments=10% | |

La densité de brillance a été mesurée selon la méthode décrite ci-dessus.

Ces résultats montrent en particulier l'avantage surprenant que procure le choix du Polydextrose comme agent auxiliaire, ainsi que l'importance de la valeur du rapport P1/P2 devant être inférieur à 3.

## Revendications

1. Composition d'enrobage (C) comprenant pour 100% de sa masse :
- de 10% à 90% massique d'un copolymère greffé de l'alcool polyvinylique et de polyéthers (P₁), et
- de 90% à 10% massique d'un agent auxiliaire d'enrobage,
**caractérisée en ce qu'**une proportion massique non nulle dudit agent auxiliaire est le polydextrose et **en ce que** le rapport massique entre le copolymère greffé (P₁) et le polydextrose est inférieur ou égal à 3.

2. Composition d'enrobage (C) telle que définie à la revendication 1, **caractérisée en ce qu'**elle comprend en outre de 2% à 30% massique d'un agent plastifiant ou d'un mélange d'agents plastifiants.

3. Composition d'enrobage (C) telle que définie à l'une des revendications 1 ou 2, **caractérisée en ce qu'**elle comprend en outre de 2% à 30% massique d'un ou plusieurs agent de coloration.

4. Composition d'enrobage (C) telle que définie à l'une des revendications 1 à 3, **caractérisée en ce qu'**elle comprend en outre de 2% à 30% massique d'une ou plusieurs charges inertes.

5. Composition d'enrobage (C) telle que définie à l'une des revendications 1 à 4, **caractérisée en ce qu'**elle comprend en outre de 1% à 20% massique d'un agent facilitant l'écoulement.

6. Composition d'enrobage (C) telle que définie à l'une des revendications 1 à 5, caractérisée en que le rapport massique entre le copolymère greffé (P₁) et le polydextrose est inférieur ou égal à 3/2.

7. Composition d'enrobage (C) telle que définie à l'une des revendications 1 à 6, caractérisée en que le rapport massique entre le copolymère greffé (P₁) et le polydextrose est supérieur ou égal à 1/3, et plus particulièrement supérieur ou égal à 2/3.

8. Composition d'enrobage (C) telle que définie à l'une des revendications 1 à 7, caractérisée en que le rapport massique entre le copolymère greffé (P₁) et le polydextrose est compris entre 2/3 et 3/2.

9. Composition d'enrobage (C) telle que définie à l'une des revendications 1 à 8, **caractérisée en ce qu'**elle se présente sous la forme d'une dispersion aqueuse, d'une poudre ou de granulés prêts à l'emploi.

10. Procédé de préparation de la composition d'enrobage (C) telle que définie à l'une des revendications 1 à 9, et se présentant sous la forme d'une poudre sèche, comprenant les étapes suivantes :
une étape (a) de mélange du copolymère greffé (P₁), du polydextrose et, si nécessaire ou si désiré un ou plusieurs autres agents auxiliaires d'enrobage, un ou plusieurs agents plastifiant, un ou plusieurs agents de coloration, une ou plusieurs charges inertes et/ou un ou plusieurs agents facilitant l'écoulement,
une étape (b) de broyage du mélange issue de l'étape (a), pour former ladite composition (C).

11. Procédé de préparation d'une composition d'enrobage (C) telle que définie à l'une des revendications 1 à 9, et se présentant sous la forme de granulés prêts à l'emploi, comprenant les étapes suivantes :
une étape (a1) de mouillage du copolymère greffé (P₁) et du polydextrose, avec éventuellement si nécessaire ou si désiré un ou plusieurs autres agents auxiliaires d'enrobage, un ou plusieurs agents plastifiant, un ou plusieurs agents de coloration, une ou plusieurs charges inertes et/ou un ou plusieurs agents facilitant l'écoulement, par une solution liante, afin d'obtenir une masse humide contenant de 30 à 60% d'eau,
une étape (b1) de séchage de la masse humide obtenue à l'étape (a1), et si désiré ou si nécessaire,
une étape (c1) de calibrage de la masse séchée obtenue à l'étape (b1) pour obtenir ladite composition (C).

12. Utilisation de la composition d'enrobage (C) telle que définie aux revendications 1 à 9, pour enrober des formes solides ingérables.

## Claims

1. Coating composition (C) comprising, per 100% of its mass:
- from 10% to 90% by mass of a grafted copolymer of polyvinyl alcohol and of polyethers (P₁), and
- from 90% to 10% by mass of an auxiliary coating agent,
**characterized in that** a non-zero mass proportion of the said auxiliary agent is polydextrose and **in that** the mass ratio between the grafted copolymer (P₁) and the polydextrose (P₂) is less than or equal to 3.

2. Coating composition (C) as defined in Claim 1, **characterized in that** it also comprises from 2% to 30% by mass of a plasticizer or of a mixture of plasticizers.

3. Coating composition (C) as defined in either of Claims 1 and 2, **characterized in that** it also comprises from 2% to 30% by mass of one or more colouring agents.

4. Coating composition (C) as defined in one of Claims 1 to 3, **characterized in that** it also comprises from 2% to 30% by mass of one or more inert fillers.

5. Coating composition (C) as defined in one of Claims 1 to 4, **characterized in that** it also comprises from 1% to 20% by mass of a glidant.

6. Coating composition (C) as defined in one of Claims 1 to 5, **characterized in that** the mass ratio between the grafted copolymer (P₁) and the polydextrose is less than or equal to 3/2.

7. Coating composition (C) as defined in one of Claims 1 to 6, **characterized in that** the mass ratio between the grafted copolymer (P₁) and the polydextrose is greater than or equal to 1/3 and more particularly greater than or equal to 2/3.

8. Coating composition (C) as defined in one of Claims 1 to 7, **characterized in that** the mass ratio between the grafted copolymer (P₁) and the polydextrose is between 2/3 and 3/2.

9. Coating composition (C) as defined in one of Claims 1 to 8, **characterized in that** it is in the form of an aqueous dispersion, a powder or ready-to-use granules.

10. Process for preparing the coating composition (C) as defined in one of Claims 1 to 9, which is in the form of a dry powder, comprising the following steps:
a step (a) of mixing the grafted copolymer (P₁), the polydextrose and, if necessary or if desired, one or more other auxiliary coating agents, one or more plasticizers, one or more colouring agents, one or more inert fillers and/or one or more glidants,
a step (b) of grinding the mixture obtained from step (a) to form the said composition (C).

11. Process for preparing a coating composition (C) as defined in one of Claims 1 to 9, which is in the form of ready-to-use granules, comprising the following steps:
a step (a1) of wetting the grafted copolymer (P₁) and the polydextrose, optionally with, if necessary or if desired, one or more other auxiliary coating agents, one or more plasticizers, one or more colouring agents, one or more inert fillers and/or one or more glidants, with a binder solution, in order to obtain a wet mass containing from 30% to 60% water,
a step (b1) of drying the wet mass obtained in step (a1), and, if desired or if necessary,
a step (c1) of calibrating the dried mass obtained in step (b1) to obtain the said composition (C).

12. Use of the coating composition (C) as defined in Claims 1 to 9, for coating ingestible solid forms.

## Patentansprüche

1. Beschichtungszusammensetzung (C), umfassend, bezogen auf 100% ihrer Masse:
- 10 bis 90 Masse-% eines Pfropfcopolymers von Polyvinylalkohol und Polyethern (P₁) und
- 90 bis 10 Masse-% eines Beschichtungshilfsmittels,
**dadurch gekennzeichnet, daß** ein von null verschiedener Massenanteil des Hilfsmittels Polydextrose ist und das Massenverhältnis zwischen dem Pfropfcopolymer (P₁) und der Polydextrose kleiner gleich 3 ist.

2. Beschichtungszusammensetzung (C) nach Anspruch 1, **dadurch gekennzeichnet, daß** sie außerdem 2 bis 30 Masse-% eines Weichmachers oder einer Mischung von Weichmachern umfaßt.

3. Beschichtungszusammensetzung (C) nach einem der Ansprüche 1 oder 2, **dadurch gekennzeichnet, daß** sie außerdem 2 bis 30 Masse-% eines oder mehrerer Farbmittel umfaßt.

4. Beschichtungszusammensetzung (C) nach einem der Ansprüche 1 bis 3, **dadurch gekennzeichnet, daß** sie außerdem 2 bis 30 Masse-% eines oder mehrerer inerter Füllstoffe umfaßt.

5. Beschichtungszusammensetzung (C) nach einem der Ansprüche 1 bis 4, **dadurch gekennzeichnet, daß** sie außerdem 2 bis 20 Masse-% eines oder mehrerer Gleitmittel umfaßt.

6. Beschichtungszusammensetzung (C) nach einem der Ansprüche 1 bis 5, **dadurch gekennzeichnet, daß** das Massenverhältnis zwischen dem Pfropfcopolymer (P₁) und der Polydextrose kleiner gleich 3/2 ist.

7. Beschichtungszusammensetzung (C) nach einem der Ansprüche 1 bis 6, **dadurch gekennzeichnet, daß** das Massenverhältnis zwischen dem Pfropfcopolymer (P₁) und der Polydextrose größer gleich 1/3 und spezieller größer gleich 2/3 ist.

8. Beschichtungszusammensetzung (C) nach einem der Ansprüche 1 bis 7, **dadurch gekennzeichnet, daß** das Massenverhältnis zwischen dem Pfropfcopolymer (P₁) und der Polydextrose zwischen 2/3 und 3/2 liegt.

9. Beschichtungszusammensetzung (C) nach einem der Ansprüche 1 bis 8, **dadurch gekennzeichnet, daß** sie in Form einer wäßrigen Dispersion, eines Pulvers oder eines gebrauchsfertigen Granulats vorliegt.

10. Verfahren zur Herstellung einer Beschichtungszusammensetzung (C) nach einem der Ansprüche 1 bis 9, die in Form eines trockenen Pulvers vorliegt, mit den folgenden Schritten:
einem Schritt (a) des Mischens des Pfropfcopolymers (P₁), der Polydextrose und, falls erforderlich oder gewünscht, eines oder mehrerer anderer Beschichtungshilfsmittel, eines oder mehrerer Weichmacher, eines oder mehrerer Farbmittel, eines oder mehrerer inerter Füllstoffe und/oder eines oder mehrerer Gleitmittel,
einem Schritt (b) des Zerkleinerns der Mischung aus Schritt (a) zur Bildung der Zusammensetzung (C).

11. Verfahren zur Herstellung einer Beschichtungszusammensetzung (C) nach einem der Ansprüche 1 bis 9, die in Form eines gebrauchsfertigen Granulats vorliegt, mit den folgenden Schritten:
einem Schritt (a1) des Befeuchtens des Pfropfcopolymers (P₁) und der Polydextrose, gegebenenfalls, falls erforderlich oder gewünscht, mit einem oder mehreren anderen Beschichtungshilfsmitteln, einem oder mehreren Weichmachern,
einem oder mehreren Farbmitteln, einem oder mehreren inerten Füllstoffen und/oder einem oder mehreren Gleitmitteln, mit einer Bindemittellösung zum Erhalt einer feuchten Masse, die 30 bis 60% Wasser enthält,
einem Schritt (b1) des Trocknens der in Schritt (a1) erhaltenen feuchten Masse und, falls gewünscht oder erforderlich,
einem Schritt (c1) des Klassierens der in Schritt (b1) erhaltenen getrockneten Masse zum Erhalt der Zusammensetzung (C).

12. Verwendung der Beschichtungszusammensetzung (C) nach einem der Ansprüche 1 bis 9 zum Beschichten von einnehmbaren festen Formen.
